# EUROPEAN PATENT APPLICATION

(11) **EP 2 253 266 A1**
(43) Date of publication of application: **24.11.2010**
(21) Application number: 09160943.8
(22) Date of filing: 22.05.2009
(51) Int. Cl.: A61B 5/00, A61K 31/00, A61K 31/519

(54) **Method for treating patients with cerebral calcifications**

(71) Applicant: Assistance Publique - Hôpitaux de Paris, 75004 Paris (FR)
(72) Inventor: Sedel, Frédérik, 75020 Paris (FR)
(74) Representative: Flesselles, Bruno F.G.

(57) **Abstract**

The invention relates to a method for assisting diagnosis and choice of treatment of patients presenting cerebral calcifications, by measuring the folate level in their cerebrospinal fluid.

## Description

The invention relates to a method for assisting diagnosis and choice of treatment of patients presenting cerebral calcifications, by measuring the folate level in their cerebrospinal fluid.

Folic acid is an essential vitamin required for key biosynthetic and epigenetic processes. Folate absorption in the small intestine depends mostly on the proton coupled folate transporter (PCFT) (Qiu et al. Cell 2006;127:917-28). In contrast, the passage of 5-methyltetrahydrofolate (5-MTHF) across choroid plexus epithelial cells into the cerebrospinal fluid (CSF) compartment involves both PCFT and the folate receptor, FR1 (Zhao et al., Blood 2007;1 10:1147-52). In addition, a low-affinity reduced folate carrier (RFC1) is expressed ubiquitously but plays only a minor role for folate transport at physiologic concentrations. Mutations of the PCFT gene are responsible for congenital folate malabsorption that results in low blood and CSF folate levels (Qiu et al. Cell 2006;127:917-28; Zhao et al., Blood 2007;110:1147-52). No human disease associated with mutations in the FR1 coding gene, named *FOLR1*, or in the RFC1 coding gene has been reported to date.

Infantile-onset cerebral folate deficiency (CFD) syndrome has been described in children with an early onset encephalopathy or with low-functioning autism with neurological deficits that respond to folinic acid supplementation (Ramaekers and Blau. Dev Med Child Neurol 2004;46:843- 51; Ramaekers et al., Neuropediatrics. 2007;3 8:276-81). Most of these children have autoantibodies of the blocking type against FR1 in their serum that impair 5-MTHF transport into the CSF compartment (Ramaekers et al., Neuropediatrics. 2007;3 8:276-81; Ramaekers et al., N Engl J Med 2005;352:1985-91).The beneficial effect of exogenously administered reduced folates despite the presence of autoantibodies, might be explained by passive diffusion or transport through RFC1 that starts to function as soon as high blood folate concentrations are reached.

Adult-onset bilateral striopallidodentate calcinosis (IBGC MIM 213600), also known as idiopathic basal ganglia calcification (IBGC), is characterized by the accumulation of calcium deposits in different brain regions, particularly in the basal ganglia. The disorder is associated with a neurodegenerative phenotype that begins between 30 and 50 years of age. The changes seen in IBGC occur in the absence of calcium or parathyroid hormone (PTH; 168450) metabolic disorders, such as hypoparathyroidism (see 146200) or pseudohypoparathyroidism (PHP; see 103580).

Idiopathic calcinosis of the basal ganglia is frequently referred to as 'Fahr disease' or 'Fahr syndrome'. Fahr (Zbl. Allg. Path. 50: 129-133, 1930) reported a sporadic case of an 81-year-old man with a long history of dementia who may have died from tetanic seizures secondary to hypoparathyroidism (Klein and Vieregge, Neurology 50: A59 only, 1998). Although some cite the postmortem examination of Fahr as showing calcifications in the basal ganglia, dentate nucleus, and cerebral cortex, Klein and Vieregge (*op. cit*.) stated that the patient had little calcification of the basal ganglia, and that calcification was primarily seen in brain vessels of the white matter. Nevertheless, Fahr's name has become associated with all forms of bilateral calcifications in the basal ganglia and other parts of the brain, including those resulting from disorders of calcium homeostasis, and is thus used to designate diseases such as 'familial idiopathic basal ganglia calcifications,' 'bilateral striopallidodentate calcinosis,' and 'idiopathic nonarteriosclerotic intracerebral calcifications.

The inventors have now demonstrated that some patients affected with a condition comprising presence of cerebral calcifications, such as the ones cited above, also present Cerebral Folate Deficiency and can be treated with reduced folate, thereby leading to improvement of their condition.

This also gives a new exploration way (search for deficit in folate in the CSF) for physicians when presented with patients with symptoms linked to presence of cerebral calcifications and / or clinical data (such as neuroimaging) demonstrating such disorders.

The result obtained by the inventors is unexpected, as Shakibai et al (Psychosomatics; 46:569-572, 2005) have indicated that level of folate were normal in a patient presenting calcification of the basal ganglia, although it is not known whether these authors looked for folate level in the blood or in the CSF.

The invention thus relates, in a first embodiment, to a method for assisting diagnosis and treatment of a patient presenting a condition comprising presence of cerebral calcifications, comprising the steps of:
a) measuring the level of 5-methyltetrahydrofolate (5-MTHF) in the cerebrospinal fluid (CSF) of said patient.

In a specific embodiment, the level of 5-MTHF may be comparing to values observed from patients without neurological diseases. This makes it possible to confirm that the measured level is indeed lower than what is normally expected.

It is considered that there is a deficiency in folate (CFD) when the measured level of 5-MTHF in the CSF is lower than 40 nmol / L. In particular, this level can be as low as 8 nmol / L and is usually in the order of 25 nmol / L ± 10 nmol / L.

References values of 5-MTHF in CSF and serum are as follows (Blau and Opladen, 2008, Folates. Laboratory Guide to the Methods in Biochemical Genetics. N. Blau, M. Duran and K. M. Gibson. Berlin Heidelberg, SpringerVerlag: 717-724)

**Table 1. Reference cerebrospinal fluid values for 5-MTHF**

| Age (years) | 5MTHF (nmol/l) |
|---|---|
| 0-0.49 | 64-182 |
| 0.5-1.99 | 64-182 |
| 2-4.99 | 63-111 |
| 5-10.99 | 41-117 |
| 11-15.99 | 41-117 |
| > 16 | 41-90 |

**Table 2. Reference serum fluid values for 5-MTHF**

| Age (years) | 5MTHF (nmol/l) |
|---|---|
| 0-0.49 | 38-90 |
| 0.5-1.99 | 36-96 |
| 2-4.99 | 22-71 |
| 5-10.99 | 14-77 |
| 11-15.99 | 12-63 |
| > 16 | 10-39 |

In a specific embodiment, the claimed method also comprises the step of harvesting some cerebrospinal fluid from said patient prior to step a), in particular by lumbar puncture.

The 5-MTHF level may be measured by any method known in the art. It is measured in particular by the method described by Blau and Opladen (2008, *op*. *cit*.), the content of which is incorporated by reference, with regard to the method of measurement of 5-MTHF.

Briefly, 5-MHTF is separated from other folates by reverse-phase HPLC and quantified with electrochemical detection. Serum is extracted with Sep-Pak C18 cartridges (Waters Chromatography) (2/5 serum mixed with 3/5 ice-cold ascorbic acid (28 µmol / l). The cartridges are activated with 2 ml methanol and 10 ml of 14 µmol/l ascorbic acid solution before the mixture is applied. After washing the cartridge with 3 ml of 14 µmol/l ascorbic acid solution, 5-MTHF is eluted with 1.5 ml methanol in a light-protected tube. The eluates are evacuated under vacuum (Speed Vac, SVC 100, Savant) and dried samples are redissolved in 100 µl of 14 µmol/l ascorbic acid solution.

CSF may be injected without pretreatment. CSF contaminated with blood should be centrifuged.

### Instrumentation (HPLC)

### CSF system

Modular isocratic HPLC with ED detection: HPLC pump 515 (Waters), Injector 7725i (Rheodyne), Pulsation Reduced (Portman Instruments), Coulochem Detector Model 5100A (ESA), Analytical Cell 5011 (ESA), and PC with chromatography software (SCPA).

| | | |
|---|---|---|
| 1. Analytical column: ODS1, 250 × 4.6 mm, 5 µm (Stagroma). | | |
| 2. Eluent: 5 mM sodium acetate. | | |
| 3. Flow: 1 ml/min. | | |
| 4. Detector 1 settings: | Potential (V) | 0.20 |
| | Polarity | - |
| | Gain | 0 × 0 |
| | Response time | 2 s |
| | Autozero | off |
| | Digital display | - |
| 5. Detector 2 settings: | Potential (V) | 0.00 |
| | Polarity | + |
| | Gain | 10 × 10 |
| | Response time | 2 s |
| | Autozero | OUT |
| | Digital display | CH 2 OUT |

Serum System

HPLC system Gold 128 (Beckman-Coulter) with autosampler (Midas, Spark) and EC detection CLC-100 (Chromsystems, Munich, Germany) is used. Sample results are analyzed by 32 Karat Software (Beckman Coulter).
1. Analytical column: Spherisorb ODS-1, 5 µm, 250 mm × 4.6 mm (Stagroma).
2. Precolumn: Spherisorb C8, 5 µm, 40 mm × 4.6 mm (Stagroma).
3. Eluent: 50 mM sodium acetate.
4. Flow: 1 ml/min.
5. EC detector settings: oxidation mode, potential of + 300 mV.

Run CSF system for 2-4 h before the first injection. Keep samples on ice. Inject 10 µl of CSF.

Calculation by external standard method.

Performance
- Linearity: 3-300 nmol/l (CSF) or 5-1600 nmol/l (serum).
- Detection limit: 3 nmol/l (CSF) or 4.5 nmol/l (serum).

Calibration mixtures may be obtained as follows:
200 µM 5MTHF: dissolve 10.0 mg 5MTHF-Ca in 100 ml of 0.05% ascorbic acid. Store 1 ml aliquots covered with Freon at -20°C. Stable for at least 1 year.
100 nM 5MTHF (CSF): dilute 100 µl of 200 µM 5MTHF solution with 200 ml of 50 mM sodium acetate. Store 1 ml aliquots covered with Freon at -20°C. Stable for at least 1 year.

6.25-1600 nM 5MTHF (serum): use 200 µM 5MTHF standard to prepare calibration standards (6.25, 12.5, 25, 50, 400, 800, 1600 nmol) by diluting with 50 mM sodium acetate. Store on ice.

When CFD is diagnosed, it may be interesting to also search for autoantibodies against the folate receptor, by any method known in the art, such as the one described in Ramaekers et al., (N Engl J Med;352:1985-91, 2005), which is a modification of Rothenberg et al., (N Engl J Med;350:134-42, 2004). Briefly, the procedure for identifying autoantibodies against membrane-bound folate receptors comprises a first incubation of the serum with solubilized purified folate receptors and then addition of [³H] folic acid. Blocking autoantibodies, if present, prevent the binding of [³H] folic acid to folate receptors. The free [³H] folic acid is removed by adsorption to dextran-coated charcoal, and the amount of receptor-bound radioactivity is measured in the supernatant (it is inversely related to the titer of the blocking autoantibodies and is expressed as picomoles of receptor blocked from binding [³H] folic acid, normalized to 1 ml of the serum assayed.

In a second embodiment, the invention relates to the use of reduced folate for the preparation of a medicament for treatment of a patient presenting a condition comprising presence of cerebral calcifications.

The invention also relates to a method of treatment of a patient presenting a condition comprising presence of cerebral calcification, wherein reduced folate is administered to said patient.

The invention also relates to the use of reduced folate for treatment of a patient presenting a condition comprising presence of cerebral calcifications, as well as to reduced folate for treating a patient presenting a condition comprising presence of cerebral calcifications.

The treatment according to the invention may be performed as a therapeutic choice to patient even if the level of 5-MTHF in the CSF has not been measured. A response to this treatment would *a posteriori* demonstrate CFD.

Nevertheless, it is preferable to first determine whether or not the patient is susceptible to respond to this type of treatment, to measure the level is 5-MTHF in the CSF and to performed this treatment when the level of 5-MTHF in the CSF of said patient is lower than 40 nmol / L.

Various drugs are usable for treating CFD. In particular, one can use calcium folinate (5-formyltetrahydrofolic acid), or the physiological product 5-methyltetrahydrofolate (5-MTHF). This product is preferred, as "*it is the naturally circulating and metabolically active folate compound in humans and was demonstrated to have high bio-availability. Administration of 5-MTHF may circumvent many genetic polymorphisms or deficiencies of enzymes, which are necessary to convert other pharmacologically available folate compounds (folic acid, 5-formyltetrahydrofolate) to the metabolically active 5-MTHF form.* [...] *5-MTHF is further the only folate form known to cross the blood-brain and blood-CSF barriers and 5MTHF toxicity studies have been found to have no adverse effects*." (as mentioned in WO 2006/119589).

Any various compositions of reduced folates, as the ones mentioned in WO 2006/119589 may be used in the context of the invention.

It is generally accepted to administer said reduced folate at a dose comprised between 0.5 mg / kg / day and 2mg / kg / day.

The reduced folate may be administered in combination with other drugs, such as L-dopa, corticosteroids (such as methylprednisolone) and the like.

In particular, said treatment also comprises intravenous injection of intravenous immunoglobulins, notably when autoantibodies against the folate receptor have been detected.

Intravenous immunoglobulin (IgIV) is a blood product administered intravenously, containing the pooled IgG immunoglobulins (antibodies) extracted from the plasma of multiple blood donors. It is mainly used as treatment in for Immune deficiencies, or inflammatory and autoimmune diseases.

As indicated above, in the treatment described in the invention, said patient harbors cerebral calcifications. Said patient may have been diagnosed with Fahr's syndrome. In general, symptoms of the disorder may include deterioration of motor function, dementia, seizures, headache, dysarthria (poorly articulated speech), spasticity (stiffness of the limbs) and spastic paralysis, eye movement impairments, and athetosis (involuntary, writhing movements). Cerebral calcification can also include symptoms characteristic of Parkinson's disease such as tremors, muscle rigidity, a mask-like facial appearance, shuffling gait, and a "pill-rolling" motion of the fingers, although these symptoms generally occur later in the development of the disease. More common symptoms include dystonia (abnormal movement caused by sustained muscle contraction) and chorea (involuntary, rapid, jerky movements).

### FIGURE LEGENDS

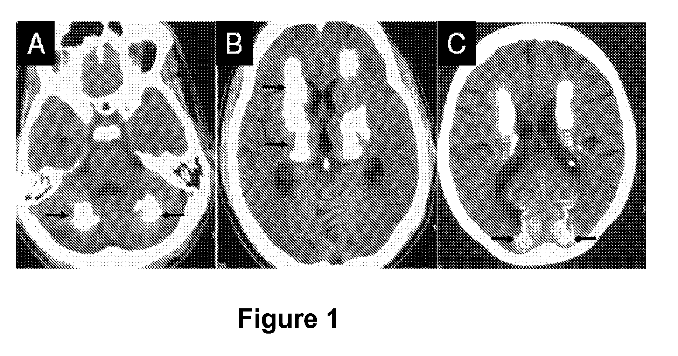

Figure 1: Brain computed tomography in patients 1 (A, B) and 2 (C) showing diffuse calcifications (arrows) of dentate nuclei (A), basal ganglia and deep white matter (B), and cortex (C).

### EXAMPLES

### Methods Patients

Three patients having diffuse cerebral calcifications involving the basal ganglia, cerebellar dentate nuclei, white matter and in one patient the occipital cortex (Table 3) were studied. These neuroradiological findings were indistinguishable from Fahr's Syndrome (IBGC MIM # 114100, # 213600).

Other investigations: The following investigations were normal for all patients: nerve conduction studies, ophthalmological examination, serum folate, vitamin B12 and homocysteine, blood cell count, mean erythrocyte corpuscular volume, calcium, phosphate, parathyroid hormone, lactate and pyruvate in blood and CSF, plasma copper and ceruloplasmin, amino acid- and organic acids, hexosaminidases and β-galactosidase. Muscular biopsy with respiratory chain analysis in patients # 1 was found to be normal.

5MTHF measurement: 5-MTHF in the CSF was measured as described in Blau and Opladen (2008, *op. cit*.) in patients # 1 and # 3 (no CSF available before treatment for patient # 2) and compared to values for 29 adults without neurological disorders (autistic features, psychiatric, behavioral problems and phenylketonuria) and for 24 patients with various neurological disorders (spastic paraparesis of unknown cause (1 case), motor neuron disease (3), degenerative dementia (3), atypical Parkinsonism (4), leukoencephalopathy of unknown cause (2), generalized dystonia caused by PLA2G6 mutations (1), SPG11 spastic paraparesis (1), episodic ataxia (1), antiphospholipids syndrome (1), Wernicke encephalopathy (1), stroke (1), multiple sclerosis (1) and normal pressure hydrocephalus (2)).

FR autoantibodies: The presence of blocking autoantibodies against the folate receptors was investigated using previously described methods (Ramaekers et al., N Engl J Med 2005;352:1985-91). Analysis was performed on the serum of all patients as well as on previously collected sera from 30 French blood donors and from 34 French patients with auto-immune diseases including Sjögren's syndrome (4 patients), anti-MAG polyneuropathy (10), rheumatoid arthritis (10), lupus erythematosus (10). The CSF of one patient with generalized dystonia and low folate in CSF (patient # 2, table 3) was also assayed.

Search for other auto antibodies: the three patients with positive FR autoantibodies and low CSF folate were also tested for the presence of other autoantibodies using standard methods that included antinuclear, anti-dsDNA, anti-Ro/SS-A, anti-La/SS-B, anti-RNP, anti-Sm, anti-cardiolipin (IgG and IgM isotypes), anti-β2 glycoprotein I (IgG and IgM isotypes), anti-neutrophilic cytoplasmic, anti-mitochondrial antibodies and rheumatoid factor.

Sequencing of the FOLR1 gene: Direct sequencing of the coding region of the adult folate receptor 1 gene (FOLR1, MIM# 136430) was performed using standard primers and conditions.

### Results

Measurement of 5-MTHF and neurotransmitters in the CSF showed low 5-MTHF in all patients compared to values obtained in 29 adults with non neurological disorders (40 to 91 nmol/L; mean±SD = 58.5±13.2 nmol/L; p-value = 2.4e-07 ) and compared to values obtained in 24 adults with various neurological diseases (34 to 113 nmol/L; mean±SD = 63.8±17.8 nmol/L; p-value = 3.87e-06 ). Homovanillic acid (HVA), a metabolite of dopamine, and the serotonin metabolite 5-hydroxyindoleacetic acid (5HIAA) were decreased in patient 1 (Table 3).

**Table 3: Clinical/radiological/biological characteristics of patients before treatment with reduced folate**

| Patient, sex, age | Disease onset | Permanent dystonia | MTHF nmol/l | HVA nmol/l | 5HIAA nmol/l | FRAb pmol/ml | Global Score |
|---|---|---|---|---|---|---|---|
| #1, M, 33 | 16 yr Progressive generalized dystonia | +++ | 8 | 95 | 47 | 1.19 | 3 |
| #2, F, 51 | 44 yr: Gait ataxia, dystonia, dysarthria | ++ | 27.8 | 175 | 79.1 | 1.48 | 3 |
| #3, F, 60 | 51 yr ataxia, diplopia | ++ | 32 | 244 | 155 | 1.04 | 3 |

| | | | | | | | |
|---|---|---|---|---|---|---|---|
| Notation +++: severe/generalized, interfering with activities of daily living, ++: obviously present, does not interfere with activities of daily living, +: present at physical examination but not disabling, 0: absent. | | | | | | | |

### Global disability score:

1: Autonomous for all activities of daily living
2: can walk at least 100 meters alone, needs help for few activities of daily living
3: can walk unaided about 10 meters, needs help for many activities of daily living
4: can not walk alone, needs aid for most activities of daily living.

### Abbreviations:

5HIAA: 5 hydroxy indole acetic acid (Normal values: 66-141 nmol/l);
FR Ab: Folate receptor auto antibodies;
MTHF: methyltetrahydrofolate in CSF (reference range 40-117 nmol/l);
HVA: Homovanylic acid in CSF (Normal values: 115-488 nmol/l).

Patient #1 had a past history of a putative stroke, while patient #2 had suffered a coma after aspirin intake.

All three patients had pyramidal signs present at physical examination (+), and severe/generalized dysarthria, interfering with activities of daily living (+++), Patients #2 and #3 presented obviously present cognitive dysfunction (++), while it was only detected at physical examination for patient #1 (+).

Patients #2 and #3 presented severe/generalized ataxia (+++), while no one was detected in patient #1.

Patients #1 and #3 presented overactive bladder (++), as well as patient #2 (+++).

Patient #1 and #3 presented bradykinesia (++ and +, respectively), while it was absent in patient #2.

None of the patients presented paroxysmal dystonia nor myoclonus.

Folate receptor autoantibodies were present in sera of all patients. No correlation was observed between the level of FR autoantibodies and MTHF values in CSF. FR autoantibodies were not detected in CSF. In controls, FR autoantibodies could be detected in the serum from six out of 30 adult French blood donors (range 0 to 1.14, mean±SD = 0.16±0.34); and, at significant levels (above 0.5) in 3 out of 34 adults with autoimmune diseases (range 0 to 1.37; mean±SD=0.2±0.36).

Search for other types of auto antibodies: The search for other autoantibodies in the serum from the patients was negative.

Sequencing of the FOLR1 gene did not reveal any mutation or variant in all 3 patients with CFD.

The clinical response to treatment was judged based on the patient's, patient's family, nurses and neurologist subjective evaluations, neurological examination and serial videos. A global disability score was designed to evaluate clinical severity and evolution (see table 3 legend). Details about treatment in every patient are presented in table 4.

**Table 4: Effect of treatments**

| **I** | **II** | **III** | **IV** | **V** | **VI** | **VII** |
|---|---|---|---|---|---|---|
| #1 | MTHF (2 mg/kg/d) | 3 | 2 | 2 years ½ | Calcium folinate led to marked initial impro-vement that waned after 6 months. IVIg (0.4gr/kgX5d every 6weeks) led to improvement of walking during 4 months | Levodopa 100 mg tid |
| | | | | | that waned thereafter. Baclofen Pump led to minor effect | |
| #2 | MTHF (2 mg/kg/d) | 3 | 3 | 14 months | Some stabilization. Attempt to arrest treatment with reduced folate caused severe recrudescence of dystonia | |
| #3 | MTHF (1.5 mg/kg/d) | 3 | 2 | 1 year | Calcium folinate 75 mg/d: same effect than MTHF | None |

| | | | | | | |
|---|---|---|---|---|---|---|
| Col. I: patient number; Col. II: current Therapy; Col. III: Global score before treatment; Col. IV: Global score after treatment; Col. V: Treatment duration; Col. VI: other treatments leading to less or only transient improvement; Col. VII: other treatments tried with no effect. | | | | | | |

Abbreviations: MTHF: methyltetrahydofolate, ND: not one

Following the diagnosis, some patients initially received pharmacologic doses of reduced folates in the form of calcium folinate (5-formyltetrahydrofolic acid), which was then switched to DL-5-methyltetrahydrofolate after a superior response was observed in some patients. The overall response after treatment was judged very good in two patients (# 1, and # 3), while no response to folate treatment could be observed in the other patient (# 2). However, an attempt to interrupt treatment with folate in the latter led to severe recrudescence of dystonia, meaning that the treatment was indeed effective. A patient who used a wheelchair before treatment (# 1) regained the ability to walk independently again. The clinical response appeared after a delay of about one month (# 1 and 3).

Indeed, for patient #1, the main results consisted in marked and sustained improvement of leg dystonia and walking: the patient does not use his wheelchair anymore. He can walk for few hundred meters (only few meters before treatment). Dysarthria, overactive bladder and alertness improved. It is to be noted that arrest of all treatments during one month (M18-M19) led to marked worsening of dystonia and walking.

For patient #3, treatment led to marked improvement of dysarthria and walking.

Treatment with IgIV in addition to folate supplements resulted in further clinical improvement in one patient (# 1), but this beneficial effect waned after a few months in this patient.

A new CFD syndrome with FR autoantibodies of the blocking type among unrelated adults with progressive dystonia is described. Bradykinesia and pyramidal signs progressively developed in most of the patients. Other important distinctive features included dysarthria and mild cognitive problems since early childhood or development of a mild subcortical frontal syndrome in adulthood. Less frequent signs were seizures in childhood, action myoclonus, gait ataxia, overactive bladder and diffuse cerebral calcifications predominating in the basal ganglia. Patients showed better improvement with 5-methyl-tetrahydrofolate than with 5-formyltetrahydrofolate supplements while one patient showed some amelioration following IgIV administration.

Cerebral folate deficiency (CFD), i.e. low levels of folate in CSF associated with normal folate levels in blood has been described in association with a variety of acquired and inherited conditions such as treatment with methotrexate, dihydropteridine reductase deficiency, Aicardi-Goutières syndrome, Rett syndrome and mitochondrial disorders. Most of these conditions can be responsible for cerebral calcifications as seen in our patients. However, CFD has never been described in the so called "idiopathic Fahr's syndrome."

These observations indicate that patients with the so-called "Fahr's syndrome" (cerebral calcifications) should be investigated for CSF 5-MTHF and serum FR autoantibodies. Further prospective studies may be needed to evaluate the prevalence of this syndrome, its full clinical spectrum and optimal therapeutic options.

## Claims

1. A method for assisting diagnosis and treatment of a patient presenting a condition comprising presence of cerebral calcifications, comprising the steps of:
a) measuring the level of 5-methyltetrahydrofolate (5-MTHF) in the cerebrospinal fluid (CSF) of said patient
b) and optionally comparing said measured level to values observed from patients without neurological diseases.

2. The method of claim 1, wherein Cerebral Folate Deficiency (CFD) is diagnosed when said level of 5-MTHF is lower than 40 nmol / L.

3. The method of any of claims 1 and 2, further comprising the step of harvesting some cerebrospinal fluid from said patient prior to step a).

4. The method of any of claims 1 to 3, wherein said 5-MTHF level is measured by reverse-phase HPLC.

5. The method of any of claims 1 to 4, further comprising search for autoantibodies against the folate receptor.

6. Use of reduced folate for the preparation of a medicament for treatment of a patient presenting a condition comprising presence of cerebral calcifications.

7. Use according to claim 6, wherein the level of folate in the cerebrospinal fluid of said patient is lower than 40 nmol / L.

8. Use according to any of claims 6 or 7, wherein said reduced folate is chosen in the group consisting of 5-methyltetrahydrofolate and 5-formyltetrahydrofolic acid (calcium folinate).

9. Use according to any of claims 6 to 8, wherein said reduced folate is administered at a dose comprised between 0.5 mg / kg / day and 2mg / kg / day.

10. Use according to any of claims 6 to 9, wherein said treatment also comprises intravenous injection of immunoglobulins.

11. Use according to any of claims 6 to 10, wherein said patient has been diagnosed with Fahr's syndrome.
